# EUROPEAN PATENT APPLICATION

(11) **EP 4 144 831 A2**
(43) Date of publication of application: **08.03.2023**
(21) Application number: 21787640.8
(22) Date of filing: 15.04.2021
(51) Int. Cl.: C12N 1/16, A23K 10/00

(54) **METHOD FOR OBTAINING BETA-GLUCAN FROM BAKER'S YEAST**

(30) Priority: 17.04.2020 CO 20004754
(71) Applicant: Compania Nacional De Levaduras Levapan S.A., Bogotá (CO)
(72) Inventor: ALZATE, Jose Jair, Valle del cauca (CO); OSORIO, Uberley, Valle del Cauca (CO); TRUJILLO, Alejandro, Tulua, Valle del Cauca (CO)
(74) Representative: Curell Suñol S.L.P.
(86) International application number: PCT/IB2021/053142
(87) International publication number: WO 2021/209959

(57) **Abstract**

The present invention refers to a process of obtaining β-glucan from yeast cells, which involves the initial growth of the yeast, the autolysis of said cells to obtain yeast cell walls, the alkaline treatment of these with sodium hydroxide comprising retention periods, the acid treatment of the insoluble product obtained, and the collection and handling thereof to obtain a powder product enriched in β-glucan with a fat percentage of less than 4%.

## Description

### Field of the invention

The present invention generally refers to processes for obtaining β-glucan from microorganisms such as *Saccharomyces cerevisiae* by solubility using alkaline agents.

### Technical background

β-glucans are linear homopolysaccharides of glucose of high molecular weight, where the units of glucose are linked through β-(1→3) and/or β-(1→4) bonds and can present ramifications due to the formation of β-(1→6) bonds in the linear glycosidic chain. β-glucans are found in the cell wall of various living organisms such as bacteria, yeasts, fungi, or plants, and, depending on the organism, β-glucan has a particular length and branching.

Currently, β-glucans are used as texturizing agents, emulsion stabilizers and thickeners for food products. Furthermore, β-glucans are used as dietary supplements where, depending on the source of the β-glucan, they are associated with various beneficial health effects. In particular, an association has been reported between the consumption of β-glucans from cereals and a reduction in plasma concentrations of cholesterol, in particular LDL cholesterol, and of the glycemic index. Likewise, β-glucans obtained from yeasts and fungi influence the stimulation of the immune response. (Pizarro, S., Ronco, A., Gotteland, M. (2014) "β-glucanos: ¿qué tipos existen and cuá/es are sus beneficios en the salud?" Revista chilena of nutrición, 41, 439-446)

In view of the great benefits offered by β-glucans particular to each organism, various methodologies for the extraction of such β-glucans are found in the state of the art, including hot water extraction, alkali extraction, microwave-assisted extraction, extraction with enzymes as elicitors, and acid extraction. The type of extraction varies depending on the organism from which the β-glucan is extracted, due to the structural variability that it may present, being the yeast β-glucan of great interest due to its properties as an immune stimulator. (Zhu, F., Du, B., & Xu, B. (2016). "A critical review on production and industrial applications of beta-glucans" Food Hydrocolloids, 52, 275-288)

In document WO1991007091, for example, a method of extraction is reported that comprises autoclaving the yeast in an alkali (NaOH), which produces a residue that is then acidified with acetic acid, and the residue obtained from this step is washed with organic solvents. With this method of extraction, β-glucan is obtained with a yield of 19%.

Additionally, WO1992007064 discloses a method of treating yeast residues that comprises the extraction of a yeast residue with a food grade alkaline salt (sodium bicarbonate), a subsequent treatment with an alkaline extraction agent, a subsequent step of bleaching with a food grade bleaching agent (hydrogen peroxide) or oxidizing/reducing agent and finally a step of pH adjustment with food grade acidic agent. From this process, product A is obtained, which is substantially free of whole yeast cells and has yeast shells with uncollapsed walls.

Likewise, patent US6060089A reveals a method for producing a product derived from baker's yeast which comprises 45-60% of polysaccharides, 15-20% of proteins, 16-22% of peptides and amino acids, and 3-5% of nucleic acids, which includes the steps of resuspending S. *cerevisiae* in a solution of strong acid (preferably HCl), separating the solid component obtained from this first step and resuspending it in a 25-30% NaCl solution, treating the solids obtained from this last step with alkaline solution (preferably NaOH), neutralize with HCl and collect the final solid component.

On the other hand, the patent document KR100485056B1 describes the culture of a mutant yeast strain (IS2) for the extraction of glucan oligomers, which comprises producing said specific strain in a liquid medium, recovering the yeast produced, adding sodium hydroxide to obtain the β-glucan from the cell walls, then add an enzyme that breaks down β-glucan to obtain the water-soluble oligomers of glucan.

In addition, WO2008032134 teaches a process for obtaining the β-glucan that involves the culture of the cells of baker's *S. cerevisiae,* the collection and autolysis of said cells, the treatment of the cell debris obtained from the autolysis with alkaline solution (sodium hydroxide solution), a subsequent treatment with a hot bleach (sodium hypochlorite), the addition of acid to adjust the pH of the solid fraction obtained from the previous step, and a final step of washing and spray drying to obtain the final powdered product. This document also reports an additional purification step with organic solvents to remove a higher percentage of fat from the final product.

Additionally, document US2011/0045545 refers to a method for preparing glucan and mannan from microorganisms such as yeast, which comprises the steps of treating the microorganism's cells with a protease and a mannase; separating the mixture obtained after the enzymatic treatment to obtain a light phase, comprising mannan, and a heavy phase, comprising glucan; treating the heavy phase with a base followed by an acid to obtain two second light and heavy phases; separate the phases obtained in the previous step and dry the new heavy phase to yield glucan at a percentage of between 60% and 85%.

Likewise, patent US8753668B2 discloses a method for processing yeast cells which comprises the autolysis of yeast cells at a certain temperature to release their cell walls, incubation with exogenous proteases, and subsequent treatment with amylase and/or lipase, to finally separate a fraction enriched in glucan and a fraction enriched in mannan.

Similarly, US20160333383A1 describes a method for preparing beta-D-glucan using a technology based on molecular assembly comprising the microfluidization of an enzymatic hydrolysate of the yeast cell wall and centrifugation, resuspension of the precipitate in an ionic liquid (as 1-ethyl-3-methylimidazolium acetate or 1-allyl-3-methylimidazolium chloride), the addition of ethanol and collection of the precipitate obtained in this step, a resuspension in water and subsequent collection of the supernatant, spray drying of said supernatant and finally obtaining β-glucan powder.

It can then be summarized from the aforementioned procedures for obtaining β-glucans that there are different strategies for extracting β-glucans, the extraction with alkalis being the most preferred. In these cases in which the extraction with alkalis is used, the prior art discloses the obtaining of fractions enriched in β-glucans that may present other components that are not of interest for the final product, such as fatty acids, which reduce the quality of the final product; said high contents of these fatty acids tend to form undesirable compounds in the flavor quality of the β-glucan, due to the formation of peroxides that affect both the flavor and the aroma of the final product due to rancidity.

Also, some methods employ steps of solvent purification and/or bleaching to achieve a purer final product of β-glucan, which increases the time of the procedure and the costs of production by requiring additional steps and reagents and does not guarantee obtaining a product with a reduced fat percentage.

Therefore, there is a need to continue developing alternative processes for obtaining β-glucans with a reduced fat percentage and with a reduced number of steps.

### Basic features of the invention

The present invention refers to a process of obtaining β-glucans comprising the following steps:
1. Yeast culture to obtain yeast cream
2. Cell autolysis of the yeast cream and obtention of the cell walls
3. Alkaline treatment of yeast cell walls
4. Acid treatment of the β-glucan fraction obtained in the alkaline treatment
5. Collection and handling of β-glucan product after acid treatment

In the first step, the culture and growth of the baker's strain of *Saccharomices cerevisiae* is carried out, which will later be collected, thus obtaining the yeast cream.

After this, the cells that make up the yeast cream are taken to the autolysis step, which occurs under specific temperature and pH conditions, to obtain the yeast cell walls in which β-glucan is found.

After the above, the yeast cell walls yeasts are subjected to an alkaline treatment, in which a strong base is added and then successive washings are carried out, in each of which some time is allowed for the retention of the cell walls in the alkaline phase.

Subsequently, the fraction obtained from the previous step is acidified and centrifuged to collect the fraction of interest, which then enters the collection and handling step, in which it is washed, sterilized, and dried, to obtain the final product of β-glucan.

Said product is enriched in β-glucan, which is above 80% by weight with respect to the total mass, and has a reduced amount of fat, which does not exceed 4% by weight with respect to the total mass.

### Detailed description of the invention

Below, the essential and not limiting features of the process of obtaining β-glucan that concerns the present application, and its preferred embodiments are defined in detail.

The process defined in the present application begins with the yeast culture step, which comprises: a preculture step, which comprises culturing yeast cells under controlled pH conditions between 2.5 and 5.0, and temperature between 28 and 35°C for approximately 20 hours, in order to obtain a primary yeast culture; a growth step, comprising inoculating a fermenter containing macronutrients and micronutrients with said preculture and maintaining the pH and temperature conditions described for the preculture for 20 to 30 hours; and a final growth step, which comprises inoculating a second fermenter with the product obtained in the previous step under pH conditions between 3.5 and 6.5 and a temperature between 28 and 38°C, where the fermentation medium comprises micronutrients that facilitate the subsequent rupture of cells in order to obtain free cell residue for the extraction of the β-glucan. Liquid yeast is obtained from this first step, which comprises 30% by weight of yeast cells.

The second step of the process of the present application refers to the autolysis of the cells in the liquid yeast obtained from the culture step. For this, the liquid yeast is brought to a temperature between 47 and 60°C, then between 0.3 and 6 liters of ethyl acetate are added for each ton of liquid yeast, and then the pH is adjusted to a value of between 3.5 and 7.5. The liquid yeast is then incubated for 10 to 72 hours, monitoring the temperature and pH in the solution in order to maintain the autolysis conditions within the desired ranges.

In a preferred embodiment, the autolysis is brought to a temperature between 49 and 51°C. In an even more preferred embodiment, the autolysis is brought to a temperature of 50°C. In a preferred embodiment, 3.3 liters of ethyl acetate are added for each ton of liquid yeast. In a preferred embodiment, the pH is adjusted to a value between 4.9 and 5.1. In a preferred embodiment, the autolysis time is 12 hours.

In another preferred embodiment, the autolysis is brought to a temperature of 50°C, 3.3 liters of ethyl acetate are added for each ton of liquid yeast, the pH is adjusted to a value between 4.9 and 5.1, and the autolysis time is 12 hours.

Additionally, within the autolysis step, an enzymatic hydrolysis step occurs. In one embodiment, said hydrolysis is mediated by endogenous enzymes of the yeast, which have an activity increased by the medium in which the autolysis of the yeast cells occurs.

In another embodiment, the hydrolysis is mediated by enzymes added to the medium in which the autolysis of the yeast cells occurs. For said enzymatic treatment, the medium is adjusted to a temperature between 60 and 62°C and a pH between 6.3 and 6.7, to then add between 90 and 95 mg of protease enzyme (E1) and between 90 and 95 mg of protease/peptidase enzyme (E5) per 1000L of medium, preferably 93.75 mg of enzyme E1 and 93.75 mg of enzyme E5 per 1000L of medium. The hydrolysis occurs for a period of between 6 and 10 hours, preferably 6 hours, during which the temperature and pH conditions are monitored to ensure that they remain within the desired range.

Once the yeast cell autolysis time has elapsed, sulfuric acid is added to reach a final pH of between 4.9 and 5.2. Next, a centrifugal separation process is carried out obtain, on the one hand, a liquid rich in protein that is used for the preparation of the yeast extract, and on the other hand, yeast cell walls that are collected and washed, and then subjected to a thermal process in a heat exchanger, in which they are heated to a temperature between 40 and 95°C, preferably between 85 and 90°C, in order to facilitate the extraction process of unwanted compounds at the time of performing the alkaline treatment.

From the autolysis and subsequent separation step, a cell wall product with a solid content between 12 and 16% is obtained. Preferably, the content of solids in the cell walls obtained is 14%.

The third step of the process of the present application refers to the alkaline treatment of the cell walls obtained, in which they are subjected to the action of an alkaline agent, preferably sodium hydroxide, and subsequently to a series of successive washes, where each of these stages of the step has a retention time that facilitates the extraction process of unwanted compounds.

The alkaline treatment begins with the heating of the cell walls to a temperature between 40 and 95°C. In a preferred embodiment, the yeast cell walls are heated to a temperature between 70 and 80°C.

Subsequently, sodium hydroxide is added to the yeast cell walls at a proportion of 20% by weight of the total yeast cell walls on a dry basis. After this, in some embodiments, water is added in order to dilute the unwanted compounds.

After the addition of the alkaline agent, the retention time of the cell walls begins, where the retention occurs at a temperature between 40 and 95°C during 2 to 6 hours. In preferred embodiments, the retention occurs between 85 and 90°C. In other preferred embodiments, the retention time is 6 hours.

Once the retention time has elapsed, the yeast cell walls are centrifuged to remove unwanted compounds that were released during the retention.

Next, the cell walls obtained are subjected to successive steps of washing at a temperature of between 40 and 95°C. Each wash comprises the addition of hot water to maintain the desired washing temperature, a subsequent retention time of 2 hours and centrifugation to remove the water with the unwanted products of the β-glucan fraction. In a preferred embodiment, the washing is carried out at a temperature between 85 and 90°C. In a preferred embodiment, between one and three successive washes are performed on the yeast cell walls. In an even more preferred embodiment, two successive washes are performed on the yeast cell walls.

The fourth step of the process of the application refers to the acid treatment of the β-glucan fraction obtained in the alkaline treatment, which consists of adding an acid agent, preferably sulfuric acid, and carrying out successive washings of said fraction.

The acid treatment of the β-glucan fraction obtained in the last wash of the alkaline treatment is carried out at a temperature between 45 and 90°C. In a preferred embodiment, the acid treatment is carried out at a temperature between 85 and 90°C.

For the acid treatment, firstly, hot water is added to the β-glucan product in order to maintain the desired temperature for this step. Then, the pH is adjusted between 1.0 and 5.0 with the addition of an acidic agent, preferably sulfuric acid, and the insoluble product of β-glucan is collected by centrifugation. In a preferred embodiment, the value of the pH is adjusted between 2.8 and 2.9.

Subsequently, several washes are carried out on the insoluble product of β-glucan collected with hot water, keeping the system at the desired temperature for this step, and the insoluble product is collected in each step of washing by centrifugation. In a preferred embodiment, between one and four successive washes are carried out on the insoluble product. In an even more preferred embodiment, three successive washes are carried out on the insoluble product.

The fifth step of the process of the present application consists of the collection and handling of the β-glucan product obtained in the previous step. For this, hot water is added at a temperature between 85 and 90°C and the pH of said product is adjusted to a value of 3.3 to 3.5. Subsequently, the product is centrifuged in order to concentrate it to obtaining between 10% and 15% of solids.

In some embodiments, the concentrated β-glucan product is sterilized by passing through a heat exchanger at a temperature between 95 and 110°C.

An antioxidant, preferably ascorbic acid, is added to the concentrated β-glucan product to reach a final concentration of between 0.01 to 1.20% by weight with respect to the volume of the β-glucan product. In a preferred embodiment, ascorbic acid is added to a final concentration of 0.075% by weight with respect to the volume of the product of β-glucan.

Finally, the β-glucan product undergoes a drying process with hot air to reduce the moisture content of the product to a 4 - 6%, thus obtaining the final β-glucan powder product.

### Example of the invention

The following is an execution example of a preferred embodiment of the process of obtaining β-glucan of the present application, which is presented for illustrative purposes and in no way limits the scope of the present disclosure.

Said preferred embodiment of the process of obtaining β-glucan of the present application, is executed under the following conditions:

| **Step** | **Step conditions** | |
|---|---|---|
| Cell autolysis | - Volume ethyl acetate: 3.3 L/ton | |
| | - Temperature: 50°C | |
| | - pH: 4.9-5.1 | |
| | - Incubation time: 12 hours | |
| | - Hydrolysis (enzymatic treatment with E1 and E5) | |
| | | ∘ Temperature: 60-62°C, |
| | | ∘ pH: 6.3-6.7 |
| | | ∘ Hydrolysis time: 6 hours |
| | - Wash and final treatment | |
| | | ∘ pH: 4.9-5.1 |
| | | ∘ Temperature: 85-90°C |
| Alkaline treatment | - Temperature: 70-80°C | |
| | - Alkaline agent: sodium hydroxide at 20% of cell wall dry basis | |
| | - Retention time: 6 hours | |
| | - Washes | |
| | | ∘ Number of washes: 2 |
| | | ∘ Temperature: 85-90°C |
| | | ∘ Retention time: 2 horas |
| Acid treatment | - Temperature: 85-90°C | |
| | - Acid agents: sulfuric acid | |
| | - pH: 2.8-2.9 | |
| | - Washes | |
| | | ∘ Number of washes: 3 |
| | | ∘ Temperature: 85-90°C |
| Collection and handling | - pH: 3.3-3.5 | |
| | - Temperature: 85-90°C | |
| | - Final concentration of ascorbic acid: 0.075% w/v | |

From the process carried out under the conditions listed above, a β-glucan product is obtained with a β-glucan percentage greater than 80%, a fat percentage less than 4%, a protein percentage less than 3.5%, an ash percentage less than 3%, and a moisture content between 4 and 6 %.

The process of obtaining β-glucan as described herein allows obtaining a β-glucan powder product with a low-fat percentage, without requiring additional bleaching or purification steps, for example, with organic solvents.

## Claims

1. A process for obtaining a β-glucan product from *Saccharomyces cerevisiae,* comprising the steps of:
a. Yeast cell culture,
b. Autolysis and enzymatic hydrolysis of the yeast cells obtained in step a,
c. Alkaline treatment of the yeast cell walls obtained in step b with sodium hydroxide and one or more water washes,
d. Acid treatment of the fraction obtained in step c, and
e. Collection and handling of the β-glucan product from step d;
wherein the sodium hydroxide is in a percentage of 20% by weight with respect to the amount of dry yeast cell wall; and wherein the washes comprise adding hot water, retaining unwanted compounds in the alkaline phase, centrifuging, and recovering the insoluble fraction, where the retention time is approximately 2 hours.

2. The process of claim 1, wherein the autolysis occurs at a temperature between 47 and 60°C, a pH between 3.5 and 7.5, and ethyl acetate is added.

3. The process of claim 1 or 2, wherein the autolysis occurs at a of 50° C.

4. The process of any one of claims 1 to 3, wherein the autolysis occurs at a pH between 4.9 and 5.1.

5. The process of any one of claims 1 to 4, wherein ethyl acetate is added at a rate of 3.3 liters per ton of liquid yeast.

6. The process of any one of claims 1 to 5, wherein the autolysis occurs for a period of 12 hours.

7. The process of claim 1 to 6, wherein the enzymatic hydrolysis occurs for 6 hours and is mediated by enzymes E1 and E5 at a temperature between 60 and 62°C and a pH between 6.3 and 6.7.

8. The process of any one of claims 1 to 7, wherein the autolysis further comprises a thermal process in a heat exchanger at a temperature between 85 and 90°C.

9. The process of any one of claims 1 to 8, wherein the alkaline treatment occurs at a temperature between 40 and 95°C and a pH between 4.9 and 5.2.

10. The process of any one of claims 1 to 9, wherein the alkaline treatment occurs at a temperature between 70 and 80°C.

11. The process of any one of claims 1 to 10, wherein the alkaline treatment comprises a retention time prior to the washes of between 2 and 6 hours.

12. The process of any one of claims 1 to 11, wherein the alkaline treatment comprises a retention time prior to the washes of 6 hours

13. The process of any one of claims 1 to 12, wherein the alkaline treatment washes occur at a temperature between 85 and 90°C.

14. The process of any one of claims 1 to 13, wherein two washes are performed in the alkaline treatment.

15. The process of any one of claims 1 to 14, wherein the acid treatment occurs at a temperature between 45 and 90°C and a pH between 1.0 and 5.0.

16. The process of any one of claims 1 to 15, wherein the acid treatment occurs at a temperature between 85 and 90°C.

17. The process of any one of claims 1 to 16, wherein the acid treatment occurs at a pH between 2.8 and 2.9.

18. The process of any one of claims 1 to 17, wherein the collection and handling of the β-glucan product occurs at a temperature between 85 and 90°C and a pH between 3.3 and 3.5.

19. The process of claim 18, further comprising a step of sterilizing the β-glucan product at a temperature between 95 and 110°C.

20. The process of any one of claims 1 to 19, wherein in the collection and handling of the β-glucan product, ascorbic acid is added at a proportion of between 0.01 and 1.20% with respect to the volume of the β-glucan product.

21. The process of any one of claims 1 to 20, wherein in the collection and handling of the β-glucan product, ascorbic acid is added at a proportion of 0.075% with respect to the volume of the β-glucan product.

22. The process of any one of claims 1 to 21, wherein the collection and handling of the β-glucan product comprises a step of drying the β-glucan product.
